# EUROPEAN PATENT APPLICATION

(11) **EP 3 235 423 A1**
(43) Date of publication of application: **25.10.2017**
(21) Application number: 15868988.5
(22) Date of filing: 26.06.2015
(51) Int. Cl.: A61B 5/00, A61B 5/01, A61B 5/103

(54) **METHOD, APPARATUS AND SYSTEM FOR PROCESSING ACQUIRED SKIN NATURE DATA**

(30) Priority: 15.12.2014 CN 201410776475
(71) Applicant: Infinitus (China) Company Ltd, Guangdong, 529100 (CN)
(72) Inventor: TANG, Jian, Jiang Men Guangdong 529100 (CN); LIU, Guangrong, Jiang Men Guangdong 529100 (CN); DENG, Wei, Jiang Men Guangdong 529100 (CN); DONG, Yinmao, Jiang Men Guangdong 529100 (CN); MENG, Hong, Jiang Men Guangdong 529100 (CN); QIU, Xianrong, Jiang Men Guangdong 529100 (CN); GAN, Xiaoshuang, Jiang Men Guangdong 529100 (CN)
(74) Representative: ABG Patentes, S.L.
(86) International application number: PCT/CN2015/082477
(87) International publication number: WO 2016/095476

(57) **Abstract**

A method, apparatus and system for processing acquired skin nature data. The method for processing acquired skin nature data comprises: acquiring vital energy and blood condition information by triggering a vital energy and blood information acquisition step (101a) in a skin nature area, acquiring skin colour condition information by triggering a skin colour information acquisition step (101b), acquiring skin elasticity information by triggering a skin nature information acquisition step (101c), acquiring skin moisture information by triggering a skin moisture information acquisition step (101d), and acquiring lustre and gloss information by triggering a skin gloss information acquisition step (101e); and comparing the acquired skin colour condition information, skin elasticity information, skin moisture information and lustre and gloss information with a pre-set index interval, and determining the general skin nature condition of the skin nature area in combination with the vital energy and blood condition information (102). The method and the corresponding apparatus and system thereof solve the technical problem in the prior art of the misjudgement of a skin nature state due to the fact that the skin nature state can be determined only by acquiring the temperature of the skin, and the most critical information data of the vital energy and blood of the skin cannot be directly acquired.

## Description

This application claims the priority of Chinese Patent Application No. 201410776475.X, filed with the Patent Office of China on December 15, 2014, titled "METHOD, APPARATUS AND SYSTEM FOR PROCESSING ACQUIRED SKIN NATURE DATA", the contents of which are incorporated herein by reference in its entirety.

### Field of the invention

The present invention relates to the field of data processing, and particularly to a method, an apparatus and a system for processing acquired skin texture data.

### Background of the invention

Life rhythm division of "female seven and male eight" in Huangdi Neijing is served as guiding ideology in the skin health maintenance system. Based on physiological characteristics of populations in different periods, they are divided into three periods and six stages, i.e. three periods of a growing period, a stable period and a degradation period, and six stages of childhood, juvenile, youth, adult, middle-age and end-age (old-age), which is referred to as "three period and six stages" health maintenance method. From the point of traditional Chinese medicine, an individual is determined by "inspection, listening/smelling, inquiry and palpation/pulse taking". The inspection is most important in skin health maintenance of the tranditional Chinese medicine. The first step in the inspection is to inspect complexion, the skin is determined and even the human health is preliminarily determined by observing the Qi and Blood change of people, essentially observing the Qi and Blood state. However, with the rapid development of science and technology, skin data are acquired, and the acquired skin data are integrated, to acquire a skin texture state. Therefore, skin health maintenance technology in the tranditional Chinese medicine tends to be scientific and digitized, so that people can directly acquire their skin state.

In the current digitized skin health maintenance technology, a human face infrared system includes a microprocessor and a display connected with a control end of the microprocessor, an infrared thermal imaging rapid temperature tester is connected to an input end of the microprocessor, so that a face temperature is tested rapidly by infrared thermal imaging rapid temperature test technology, and the obtained information on the temperature is transmitted to the microprocessor to perform qualitative and quantitative analysis and processing, and then is compared and matched with a healthy standard temperature value built in the microprocessor or disease information corresponding to a temperature value when being ill.

However, in the solution described above in which the temperature of the human face is tested rapidly by the infrared thermal imaging rapid temperature test technology, only the temperature of the skin is acquired to determine the skin texture state, and the most critical information data of the "Qi and Blood" of the skin cannot be acquired directly, thereby resulting in a technical problem of misjudgement of the skin texture state.

### Summary of the invention

A method, apparatus and system for processing acquired skin texture data are provided by the embodiments of the present invention, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired.

A method for processing acquired skin texture data are provided by an embodiment of the present invention, which includes:
acquiring Qi and Blood state information by triggering a Qi and Blood information acquisition step in a skin texture area, acquiring skin tone state information by triggering a skin tone information acquisition step in the skin texture area, acquiring skin texture elasticity information by triggering a skin texture information acquisition step in the skin texture area, and acquiring skin moisture information by triggering a skin moisture information acquisition step in the skin texture area, and acquiring luster and gloss information by triggering a skin gloss information acquisition step in the skin texture area; and
comparing the acquired skin tone state information, the acquired skin texture elasticity information, the acquired skin moisture information and the acquired luster and gloss information with preset indicator intervals, and determining the overall skin texture state of the skin texture area by combining with the Qi and Blood state information;
wherein, the Qi and Blood information acquisition step includes:
   acquiring the blood state of the skin texture area by a detection device;
   generating an image corresponding to the skin texture area based on the blood state;
   analyzing the image to obtain blood flow distribution information corresponding to the image; and
   judging whether the blood flow distribution information falls within a preset blood flow information range, and in the case that the blood flow distribution falls within the preset blood flow information range, the Qi and Blood state information of the skin texture area is determined to be normal.

Preferably, said acquiring a blood state of the skin texture area by a detection device, generating an image corresponding to the skin texture area based on the blood flow state specifically includes:
acquiring a temperature signal in the skin texture area by a thermal infrared imager, and at the same time acquiring the blood perfusion signal state in the skin texture area by a Doppler imager; and
generating the image corresponding to the skin texture area according to image processing means in combination with the temperature signal and the blood perfusion signal.

Preferably, said analyzing the image to obtain the blood flow distribution information corresponding to the image specifically includes:
analyzing the image to obtain a blood flow distribution parameter corresponding to the image, wherein the blood flow distribution information includes a temperature value and a blood perfusion amount of the skin texture area.

Preferably, the skin tone information acquisition step specifically includes:
determining the contents of heme and melanin of the skin texture area by means of narrow-band spectrum.

Preferably, the skin texture information acquisition step specifically includes:
determining the elasticity value of the skin texture area by means of interaction between suction and stretch.

Preferably, the skin moisture information acquisition step specifically includes:
determining the moisture content of the skin texture area by means of a skin moisture capacitance test, and obtaining oil content detected by an oil detecting device.

Preferably, the skin gloss information acquisition step specifically includes:
determining the luster and gloss of the skin texture area by means of a specular gloss test.

Preferably, said comparing the acquired skin tone state information, the acquired skin texture elasticity information, the acquired skin moisture information and the acquired luster and gloss information with preset indicator intervals, and determining the overall skin texture state of the skin texture area by combining with the Qi and Blood state information specifically includes:
comparing the acquired skin tone state information, the acquired skin texture elasticity information, the acquired skin moisture information and the acquired luster and gloss information with preset indicator intervals one by one, and determining the overall skin texture state of the skin texture area by combining with the Qi and Blood state information; and
the preset indicator intervals consist of a skin tone indicator, a skin texture elasticity indicator, a skin moisture indicator and a luster and gloss indicator.

Preferably, the skin texture area is a facial area.

Preferably, the blood flow distribution information includes local capillary blood flow rate and a local tissue metabolic function of the face.

An apparatus for processing acquired skin texture data are provided by an embodiment of the present invention , which includes:
a Qi and Blood acquisition module configured to acquire Qi and Blood state information by performing a Qi and Blood information acquisition step in a skin texture area;
a skin tone acquisition module configured to acquire skin tone state information by triggering a skin tone information acquisition step in the skin texture area;
a skin texture acquisition module configured to acquire skin texture elasticity information by triggering a skin texture information acquisition step in the skin texture area;
a moisture acquisition module configured to acquire skin moisture information by triggering a skin moisture information acquisition step in the skin texture area;
a gloss acquisition module configured to acquire luster and gloss information by triggering a skin gloss information acquisition step in the skin texture area; and
an analysis module configured to compare the acquired skin tone state information, the acquired skin texture elasticity information, the acquired skin moisture information and the acquired luster and gloss information with preset indicator intervals, and determine the overall skin texture state of the skin texture area by combining with the Qi and Blood state information;
wherein, the Qi and Blood acquisition module specifically includes:
   an acquisition submodule configured to acquire the blood state of the skin texture area by a detection device;
   a generation submodule configured to generate an image corresponding to the skin texture area based on the blood state;
   an analysis submodule configured to analyze the image to obtain blood flow distribution information corresponding to the image; and
   a judgement submodule configured to judge whether the blood flow distribution information falls within a preset blood flow information range, and in the case that the blood flow distribution information falls within the preset blood flow information range, the Qi and Blood state information of the skin texture area is determined to be normal.

Preferably, the skin tone acquisition module is specifically configured to determine the contents of heme and melanin of the skin texture area by triggering the skin texture area by means of narrow-band spectrum;
the skin texture acquisition module is specifically configured to determine the elasticity value of the skin texture area by triggering the skin texture area by means of interaction between suction and stretch;
the moisture acquisition module is specifically configured to determine the moisture content of the skin texture area by triggering the skin texture area by means of a skin moisture capacitance test, and obtain oil content detected by an oil detecting device; and
the gloss acquisition module is specifically configured to determine the luster and gloss of the skin texture area by triggering the skin texture area by means of a specular gloss test.

Preferably, the analysis module is specifically configure to compare the acquired skin tone state information with a preset skin tone value, compare the acquired skin texture elasticity information with a preset elasticity value, compare the acquired skin moisture information with preset moisture information, and compare the acquired luster and gloss information with preset gloss information, and in the case that the heme content increases and the melanin content decreases, the texture value of the skin decreases, the moisture content of the skin increases, and the brightness value of the skin increases, it is determined that the overall skin texture Qi and Blood state of the skin texture area is good.

A system for processing acquired skin texture data are provided by an embodiment of the present invention, which includes:
the apparatus for processing acquired skin texture data according to any one of the embodiment of the present invention; and
a thermal infrared imaging device, a skin heme and melanin test device, a skin elasticity test device, a skin moisture test device, an oil test device and a multi-function skin test device;
wherein, the thermal infrared imaging device, the skin heme and melanin test device, the skin elasticity test device, the skin moisture test device, the oil test device and the multi-function skin test device are electrically connected with the apparatus for processing the acquired skin texture data, respectively.

Preferably, the thermal infrared imaging device is configured to provide tested Qi and Blood state information to the apparatus for processing acquired skin texture data via a test probe;
the skin heme and melanin test device is configured to provide tested skin tone state information to the apparatus for processing acquired skin texture data via a test probe;
the skin elasticity test device is configured to provide tested skin texture elasticity information to the apparatus for processing acquired skin texture data via a test probe;
the skin moisture test device is configured to provide tested skin moisture information to the apparatus for processing acquired skin texture data via a test probe;
the oil test device is configured to provide tested skin oil content information to the apparatus for processing acquired skin texture data via a test probe; and
the multi-function skin test device is configured to provide tested luster and gloss information to the apparatus for processing acquired skin texture data via a test probe.

It can be seen from the technical solution described above, that the embodiments of the present invention have the advantages as follows.

A method, an apparatus and a system for processing acquired skin texture data are provided by the embodiments of the present invention. Wherein, the method includes: acquiring Qi and Blood state information by triggering a Qi and Blood information acquisition step in a skin texture area, acquiring skin tone state information by triggering a skin tone information acquisition step, acquiring skin texture elasticity information by triggering a skin texture information acquisition step, and acquiring skin moisture information by triggering a skin moisture information acquisition step, and acquiring luster and gloss information by triggering a skin gloss information acquisition step; comparing the acquired skin tone state information, the acquired skin texture elasticity information, the acquired skin moisture information and the acquired luster and gloss information with preset indicator intervals, and determining the overall skin texture state of the skin texture area by combining with the Qi and Blood state information. Wherein, the Qi and Blood information acquisition step includes: acquiring the blood state of the skin texture area by a detection device; generating an image corresponding to the skin texture area based on the blood state; analyzing the image to obtain blood flow distribution information corresponding to the image; judging whether the blood flow distribution information falls within a preset blood flow information range, and in the case that the blood flow distribution falls within the preset blood flow information range, the Qi and Blood state information of the skin texture area is determined to be normal. In the embodiments, the blood state of the skin texture is acquired by a detection device for the Qi and Blood information acquisition step, and the blood information is analyzed to obtain blood flow distribution information corresponding to the blood information, whether the blood flow distribution information falls within a preset blood flow information range is judged, to determine a blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired.

### Description of the drawings

In order to more clearly illustrate the technical solution in the embodiments of the present invention or in the prior art, the drawings required in the description of the embodiments or the prior art will be briefly introduced hereinafter. Obviously, the drawings in the following description are only some embodiments of the present invention. For those skilled in the art, other drawings can also be obtained according to these drawings without any creative work.
Figure 1 is a schematic flow diagram of a method for processing acquired skin texture data provided by an embodiment of the present invention.
Figure 2 is a schematic flow diagram of a method for processing acquired skin texture data provided by another embodiment of the present invention.
Figure 3 is a schematic flow diagram of a method for processing acquired skin texture data provided by another embodiment of the present invention.
Figure 4 is a schematic structural diagram of an apparatus for processing acquired skin texture data provided by an embodiment of the present invention; and

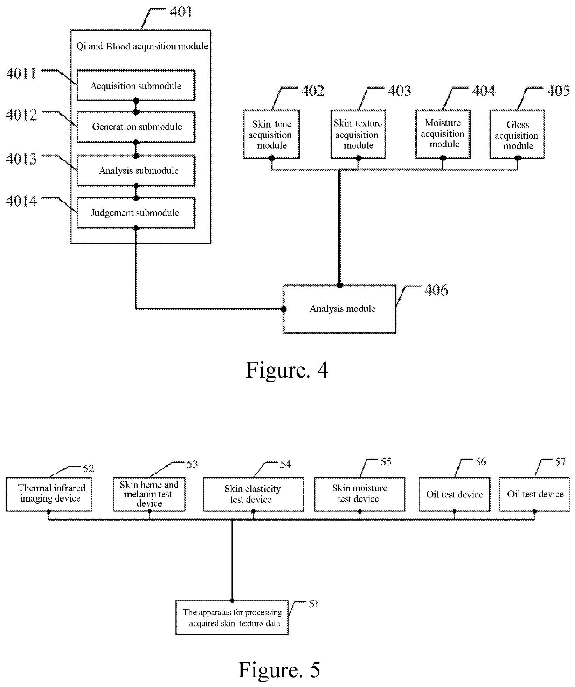

Figure 4 is a schematic structural diagram of a system for processing acquired skin texture data provided by an embodiment of the present invention.

### Detailed embodiments of the invention

A method, an apparatus and a system for processing acquired skin texture data are provided by the embodiments of the present invention, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired.

In order to make the objective, feature and advantage of the present invention clearer and easier to be understood, the technical solution according to the embodiments of the present invention is described clearly and completely in conjunction with the drawings of the embodiments of the present invention, apparently, the embodiments described below are only a part rather than all of the embodiments. Based on the embodiments of the present invention, all the other embodiments acquired by those skilled in the art without creative work fall within the protection scope of the present invention.

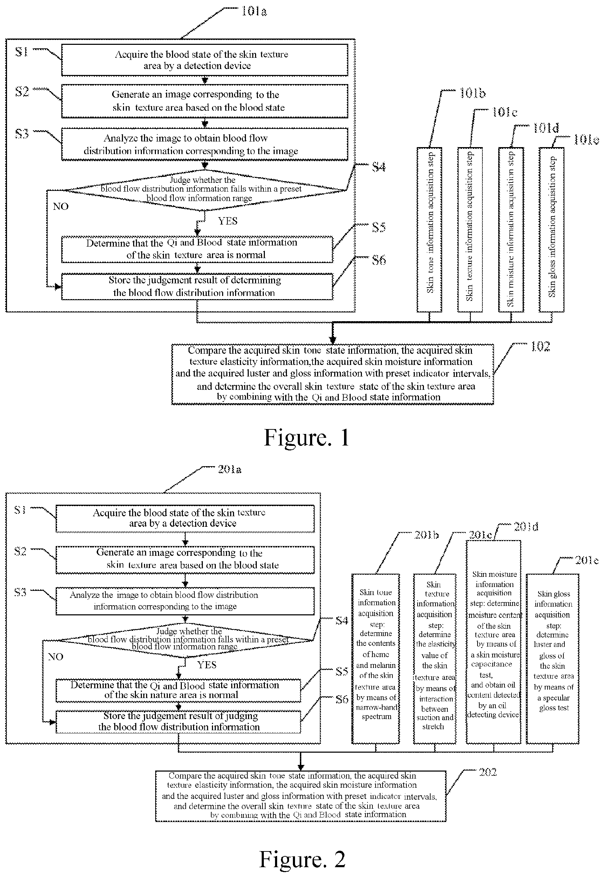

With reference to Figure 1, a method for processing acquired skin texture data provided by an embodiment of the present invention includes the stepsof:
101, Qi and Blood state information is acquired by triggering a Qi and Blood information acquisition step 101a in a skin texture area, skin tone state information is acquired by triggering a skin tone information acquisition step 101b in the skin texture area, skin texture elasticity information is acquired by triggering a skin texture information acquisition step 101c in the skin texture area, skin moisture information is acquired by triggering a skin moisture information acquisition step 101d in the skin texture area, and luster and gloss information is acquired by triggering a skin gloss information acquisition step 101e in the skin texture area.

In the embodiment, in the case that a skin texture state of a local skin texture area in a certain area is to be determined, the acquired skin texture data are processed. Firstly, the Qi and Blood state information is acquired by triggering the Qi and Blood information acquisition step 101a in a skin texture area, the skin tone state information is acquired by triggering the skin tone information acquisition step 101b in the skin texture area, the skin texture elasticity information is acquired by triggering the skin texture information acquisition step 101c in the skin texture area, the skin moisture information is acquired by triggering the skin moisture information acquisition step 101d in the skin texture area, and the luster and gloss information is acquired by triggering the skin gloss information acquisition step 101e in the skin texture area.

The Qi and Blood state information may be observation for microcirculation of Qi and Blood, including two types of isolated specimen observation and living observation. The isolated specimen observation method mainly includes microvascular perfusion, casting mould, tissue section staining and so on. Microvascular endothelial cell and the ultrastructure thereof can be precisely observed by tissue section, however, a stereo distribution and configuration of the microvessel cannot be shown. The specimen obtained after the perfusion and casting mould can show a three-dimensional configuration of the microvascular well and can measure the length, the width and the area of the microvessel. The disadvantages of the isolated specimen observation are that only microvessel of death or postoperative isolated organs can be used for observation, and the physiological state cannot be reflected. In the living observation, a blood flow speed of the microvessel is observed directly under various microscopes. Currently, there are some shortcomings in the observation method and technology, some observation methods are still invasive, for example, an observation window is installed on the animal skin to expose a subcutaneous vascular network.

The skin tone state information described above may mainly include four pigments of the human skin, that is, black-brown melanin, red oxidized hemoglobin, blue decreased hemoglobin and yellow carotene. From the prospective of skin biology, a color of the human skin is affected by the pigment system, the melanin is a product of melanocytes, content and distribution states of these pigments are major factors for determining the color of the skin. Complexion is pink under the influence of hemoglobin (hemachrome) content in the blood of the skin. Oxygenated hemoglobin is bright red, and (decreased hemoglobin) becomes dark red in hypoxia, the color of the skin changes correspondingly.

The skin texture elasticity information described above may be texture, i.e. skin texture, and may be indicated by a degree of texture, such as skin roughness or smoothness and tenderness. Surface texture of the skin is an important characteristic of skin aging. Skin texture test is to test an elasticity indicator of skin. The method used in a skin texture tester on the market includes a suction method, a torsion method and a method for measuring a transmission speed of an elastic shear wave. It should be noted that the skin aging of human includes intrinsic aging and photo-induced aging. The intrinsic aging may be regarded as a change in skin histology and physiology caused by intrinsic genetic factors. The intrinsic aging includes epidermal atrophy, dermal atrophy and flattening of an interface between epidermis and dermis and so on, which, as a performance of natural aging within humans, are irresistible but can be delayed. The photo-induced aging is caused by sunshine, which is a reason for skin keratosis, dark spots, wrinkles and elastic tissue degeneration. Abnormal skin keratosis may include epidermal thickening and densification, which make the skin dry and rough.

The skin moisture information described above may include a skin moisture degree and the oil content, and is indicated as differences in the moisture content and the oil content of the skin cuticle. Moisture is one of the important shaping materials in the cuticle of the skin epidermis, and the cuticle of the epidermis becomes thinner when the skin is aging.

The content of nature moisturizing factor in the cuticle decreases, hydratability of the skin decreases and moisture loss of the skin increases, and a histology structure and morphology change with shrinkage of the cell and atrophy of the tissue, so that small wrinkles gradually occur in the skin, and the surface area of the skin is increasing with further increasing and deepening of the wrinkles, and the epidermis become thinner, moisture loss is more serious and skin aging is aggravated. By testing the moisture of the skin, not only the moisture content in the cuticle of the skin epidermis can be determined directly, but also the extent of the skin aging can be reflected indirectly. The sheddings produced when sebaceous glands secrete oil will clog the pores, therefore, acnes occur. In contrast, sebum secretion decreases, the produced hydro-lipid emulsion decreases, which makes the skin dry, rough, dull, and developing other symptoms.

The luster and gloss information described above may include a brightness change of the skin.

It should be illustrated that processes of triggering the skin tone information acquisition step, triggering the skin texture information acquisition step, triggering the skin moisture information acquisition step and triggering the skin gloss information acquisition described above are described in detail in the embodiments below, which are not described here repeatedly anymore.

As shown in Figure 1, the Qi and Blood information acquisition step 101a includes steps of:
S1, the blood state of the skin texture area is acquired by a detection device.

In the embodiment, in the case that the Qi and Blood information is to be acquired by triggering the Qi and Blood information acquisition step 101a, the blood state of the skin texture area is firstly acquired by the detection device. It should be illustrated that the way of acquiring the blood state of the skin texture area by the detection device is described in detail in subsequent embodiments, which are not described here in detail anymore.

S2, an image corresponding to the skin texture area is generated based on the blood state.

After the blood state of the skin texture area is acquired by the detection device, a thermal image corresponding to the skin texture area is generated based on the blood state, it should be understood that, the image described above includes various technologies, such as micro-MRI, micro-CT, micro-PET (positron emission tomography), near-infrared ray (ultraviolet) fluorescence imaging, multi-photon imaging, ultrasound molecular imaging, diffuse optical tomography, optical bonding imaging, fluorescence sub-surface imaging, bioluminescence imaging and thermal imaging, which are not limited hereby.

S3, the image is analyzed to obtain blood flow distribution information corresponding to the image.

After the image corresponding to the skin texture area is generated based on the blood state, the image is analyzed to obtain blood flow distribution information corresponding to the image.

S4, whether the blood flow distribution information falls within a preset blood flow information range is judged. Step S5 is performed in the case that the blood flow distribution information falls within the preset blood flow information range, and step S6 is performed in the case that the blood flow distribution information does not fall within the preset blood flow information range.

After the image is analyzed to obtain blood flow distribution information corresponding to the image, whether the blood flow distribution information falls within the preset blood flow information range is judged. Step S5 is performed in the case that the blood flow distribution information falls within the preset blood flow information range, and step S6 is performed in the case that the blood flow distribution information does not fall within the preset blood flow information range.

S5, it is determined that Qi and Blood state information of the skin texture area is normal.

It is determined that the Qi and Blood state information of the skin texture area is normal in the case that the blood flow distribution information falls within the preset blood flow information range, and step S6 is proceeded.

S6, a judgement result of judging the blood flow distribution information is stored.

The judgement result of judging the blood flow distribution information is stored in the case that it is determined in step S4 that the blood flow distribution information does not fall within the preset blood flow information range or it is determined that the Qi and Blood state information of the skin texture area is normal.

102, the acquired skin tone state information, the acquired skin texture elasticity information, the acquired skin moisture information and the acquired luster and gloss information are compared with preset indicator intervals, and the overall skin texture state of the skin texture area is determined by combining with the Qi and Blood state information.

After the Qi and Blood state information is acquired by triggering the steps S1 to S6 of the Qi and Blood information acquisition step 101a in the skin texture area, and the skin tone state information is acquired by triggering the skin tone information acquisition step 101b in the skin texture area, the skin texture elasticity information is acquired by triggering the skin texture information acquisition step 101c in the skin texture area, and the skin moisture information is acquired by triggering the skin moisture information acquisition step 101d in the skin texture area, and the luster and gloss information is acquired by triggering the skin gloss information acquisition step 101e in the skin texture area, the acquired skin tone state information, the acquired skin texture elasticity information, the acquired skin moisture information and the acquired luster and gloss information are compared with preset indicator intervals, and the overall skin texture state of the skin texture area is determined by combining with the Qi and Blood state information.

It should be illustrated that the preset indicator intervals described above include standard data indicators acquired by technicians of the present invention through long-term experiments.

The skin texture area described in the embodiment may also be a facial area, and the blood flow distribution information described above may also include local capillary blood flow rate and a local tissue metabolic function of the face.

In the embodiment, the blood flow state of the skin texture is acquired by the detection device for the Qi and Blood information acquisition step, and the image is analyzed to obtain the blood flow distribution information corresponding to the image, whether the blood flow distribution information falls within the preset blood flow information range is judged, to determine the blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired.

The flow of the method for processing acquired skin texture data are described in detail above, processes of triggering the skin tone information acquisition step, triggering the skin texture information acquisition step, triggering the skin moisture information acquisition step and triggering the skin gloss information acquisition step are described in detail below. With reference to Figure 2, a method for processing acquired skin texture data according to another embodiment of the present invention includes steps 201 to 202.

201, Qi and Blood state information is acquired by triggering a Qi and Blood information acquisition step 201 a in a skin texture area, skin tone state information is acquired by triggering a skin tone information acquisition step 201b in the skin texture area, skin texture elasticity information is acquired by triggering a skin texture information acquisition step 201c in the skin texture area, skin moisture information is acquired by triggering a skin moisture information acquisition step 201d in the skin texture area, and luster and gloss information is acquired by triggering a skin gloss information acquisition step 201e in the skin texture area.

In the embodiment, in the case that a skin texture state of a local skin texture area in a certain area is to be determined, the acquired skin texture data are processed. Firstly, the Qi and Blood state information is acquired by triggering the Qi and Blood information acquisition step 201a in a skin texture area, the skin tone state information is acquired by triggering the skin tone information acquisition step 201b in the skin texture area, the skin texture elasticity information is acquired by triggering the skin texture information acquisition step 201c in the skin texture area, the skin moisture information is acquired by triggering the skin moisture information acquisition step 201d in the skin texture area, and the luster and gloss information is acquired by triggering the skin gloss information acquisition step 201e in the skin texture area.

The Qi and Blood state information may be observation for microcirculation of Qi and Blood, including two types of isolated specimen observation and living observation. The isolated specimen observation method mainly includes microvascular perfusion, casting mould, tissue section staining and so on. Microvascular endothelial cell and the ultrastructure thereof can be precisely observed by tissue section, however, a stereo distribution and configuration of the microvessel cannot be shown. The specimen obtained after the perfusion and casting mould can show a three-dimensional configuration of the microvascular well and can measure the length, the width and the area of the microvessel. The disadvantages of the isolated specimen observation are that only microvessel of death or postoperative isolated organs can be used for observation, and the physiological state cannot be reflected. In the living observation, a blood flow speed of the microvessel is observed directly under various microscopes. Currently, there are some shortcomings in the observation method and technology, some observation methods are still invasive, for example, an observation window is installed on the animal skin to expose a subcutaneous vascular network.

The skin tone state information described above may mainly include four pigments of the human skin, that is, black-brown melanin, red oxidized hemoglobin, blue decreased hemoglobin and yellow carotene. From the prospective of skin biology, a color of the human skin is affected by the pigment system, the melanin is a product of melanocytes, content and distribution states of these pigments are major factors for determining the color of the skin. Complexion is pink under the influence of hemoglobin (hemachrome) content in the blood of the skin. Oxygenated hemoglobin is bright red, and (decreased hemoglobin) becomes dark red in hypoxia, the color of the skin changes correspondingly.

The skin texture elasticity information described above may be texture, i.e. skin texture, and may be indicated by a degree of texture, such as skin roughness or smoothness and tenderness. Surface texture of the skin is an important characteristic of skin aging. Skin texture test is to test an elasticity indicator of skin. The method used in a skin texture tester on the market includes a suction method, a torsion method and a method for measuring a transmission speed of an elastic shear wave. It should be noted that the skin aging of human includes intrinsic aging and photo-induced aging. The intrinsic aging may be regarded as a change in skin histology and physiology caused by intrinsic genetic factors. The intrinsic aging includes epidermal atrophy, dermal atrophy and flattening of an interface between epidermis and dermis and so on, which, as a performance of natural aging within humans, are irresistible but can be delayed. The photo-induced aging is caused by sunshine, which is a reason for skin keratosis, dark spots, wrinkles and elastic tissue degeneration. Abnormal skin keratosis may include epidermal thickening and densification, which make the skin dry and rough.

The skin moisture information described above may include a skin moisture degree and the oil content, and is indicated as differences in the moisture content and the oil content of the skin cuticle. Moisture is one of the important shaping materials in the cuticle of the skin epidermis, and the cuticle of the epidermis becomes thinner when the skin is aging.

The content of nature moisturizing factor in the cuticle decreases, hydratability of the skin decreases and moisture loss of the skin increases, and a histology structure and morphology change with shrinkage of the cell and atrophy of the tissue, so that small wrinkles gradually occur in the skin, and the surface area of the skin is increasing with further increasing and deepening of the wrinkles, and the epidermis become thinner, moisture loss is more serious and skin aging is aggravated. By testing the moisture of the skin, not only the moisture content in the cuticle of the skin epidermis can be determined directly, but also the extent of the skin aging can be reflected indirectly. The sheddings produced when sebaceous glands secrete oil will clog the pores, therefore, acnes occur. In contrast, sebum secretion decreases, the produced hydro-lipid emulsion decreases, which makes the skin dry, rough, dull, and developing other symptoms.

The luster and gloss information described above may include a brightness change of the skin.

The skin tone state information, the skin texture elasticity information, the skin moisture information and the luster and gloss information will be described in detail in subsequent embodiments, which are not described in detail here anymore.

As shown in Figure 2, the Qi and Blood acquisition step 201a described above includes steps of:
S1, the blood state of the skin texture area is acquired by a detection device.

In the embodiment, in the case that the Qi and Blood information is to be acquired by triggering the Qi and Blood information acquisition step 201a, the blood state of the skin texture area is firstly acquired by the detection device. It should be illustrated that the way of acquiring the blood state of the skin texture area by the detection device is described in detail in subsequent embodiments, which are not described here in detail anymore.

S2, an image corresponding to the skin texture area is generated based on the blood state.

After the blood state of the skin texture area is acquired by the detection device, a thermal image corresponding to the skin texture area is generated based on the blood state, it should be understood that, the image described above includes various technologies, such as micro-MRI, micro-CT, micro-PET (positron emission tomography), near-infrared ray (ultraviolet) fluorescence imaging, multi-photon imaging, ultrasound molecular imaging, diffuse optical tomography, optical bonding imaging, fluorescence sub-surface imaging, bioluminescence imaging and thermal imaging, which are not limited hereby.

S3, the image is analyzed to obtain blood flow distribution information corresponding to the image.

After the image corresponding to the skin texture area is generated based on the blood state, the image is analyzed to obtain blood flow distribution information corresponding to the image.

S4, whether the blood flow distribution information falls within a preset blood flow information range is judged. Step S5 is performed in the case that the blood flow distribution information falls within the preset blood flow information range, and step S6 is performed in the case that the blood flow distribution information does not fall within the preset blood flow information range.

After the image is analyzed to obtain blood flow distribution information corresponding to the image, whether the blood flow distribution information falls within the preset blood flow information range is judged. Step S5 is performed in the case that the blood flow distribution information falls within the preset blood flow information range, and step S6 is performed in the case that the blood flow distribution information does not fall within the preset blood flow information range.

S5, it is determined that Qi and Blood state information of the skin texture area is normal.

It is determined that the Qi and Blood state information of the skin texture area is normal in the case that the blood flow distribution information falls within the preset blood flow information range, and step S6 is proceeded.

S6, a judgement result of judging the blood flow distribution information is stored.

The judgement result of judging the blood flow distribution information is stored in the case that it is determined in step S4 that the blood flow distribution information does not fall within the preset blood flow information range or it is determined that the Qi and Blood state information of the skin texture area is normal.

As shown in Figure 2, the skin tone information acquisition step 201b described above includes determining the contents of heme and melanin of the skin texture area by means of narrow-band spectrum.

In the case that the skin tone state information is to be acquired, heme and melanin contents of the skin texture area is determined by means of narrow-band spectrum, for example, a skin heme and melanin tester and a test probe (Mexameter MX18) determine the heme and melanin contents in the skin by testing the amount of reflection of light at a specific wavelength on the human skin based on the principle of spectral absorption (RGB). The emitter of the instrument probe emits light at wavelengths of 568nm, 660nm, 880nm to irradiate onto a surface of the skin, and a receiver tests the light reflected by the skin. Since that the amount of emitted light is fixed, the heme and melanin contents can be tested by testing the amount of light absorbed by the skin. The test range of the instrument ranges from 0 to 999, and the greater the tested value is, the higher the heme and melanin contents in the skin are.

As shown in Figure 2, the skin texture information acquisition step 201c described above includes determining the elasticity value of the skin texture area by means of interaction between suction and stretch.

In the case that the skin texture elasticity information is to be acquired, the elasticity value of the skin texture area is determined by means of interaction between suction and stretch. The skin elasticity tester and test probe have a test principle based on suction and tension, negative pressure is forced on the surface of the skin to be tested to suck the skin into a test probe, the depth of skin which is sucked into the test probe is tested by a non-contact optical test system. The test probe includes an optical emitter and an optical receiver, the ratio of the emitted light to the received light is in direct proportion to the depth of the skin which is sucked into the test probe, in this way, a relation curve between an extension length of the skin and time is obtained, and an elasticity performance of the skin can be determined by the curve.

As shown in Figure 2, the skin moisture information acquisition step 201d described above includes determining moisture content of the skin texture area by means of a skin moisture capacitance test, and obtaining oil content of the skin texture area detected by an oil detecting device.

In the case that the skin moisture information is to be acquired, the moisture content of the skin texture area is determined by means of a skin moisture capacitance test, and oil content of the skin texture area detected by an oil detecting device is acquired. The world recognized CORNEOMETER capacitance method is used by a skin moisture content tester and test probe (Corneometer CM 825). The principle of the CORNEOMETER capacitance method is that a dielectric constant based on moisture and other substances changes considerably, capacitance of the skin detected by a test capacitor in a proper shape changes with the moisture content, the capacitance of the skin falls within a test range, in this way, the moisture content of the skin can be tested. The result for the moisture content of the skin is represented as a set moisture measurement value (Moisture Measurement Value, MMV). The value of MMV ranges from 0 to 150. The greater the MMV value is, the higher the moisture content of the skin cuticle is. Skin moisture loss TEWL is an important parameter for assessing a function of a skin moisture protection layer, and has been widely accepted internationally. The more complete the skin moisture protection layer is, the higher the moisture content is and the lower a value of the skin moisture loss TEWL is. In other words, a change in moisture vapor pressure on the surface of adjacent skin is measured by the skin moisture loss tester and a test probe (Tewamater TM300) according to the diffusion principle. A relatively stable test microenvironment is formed on the surface of the skin by a specially designed test probe of a cylindrical cavity having two open ends, two sets of temperature sensors and moisture sensors test water vapor pressure gradient between different two points within near epidermis (approximating to 1 cm) caused by the moisture loss in the cuticle, to directly test the amount of moisture evaporated via the epidermis, thereby evaluating moisture loss on the surface of the skin. The less the value of TEWL is, the less the amount of moisture loss via the epidermis is, the unit of the amount of moisture loss is g/hm². Furthermore, according to the principle of skin oil secretion, the sheddings produced when the sebaceous glands secrete oil will clog the pores, therefore, acnes occur. In contrast, sebum secretion decreases, the produced hydro-lipid emulsion decreases, which makes the skin dry, rough, dull, and developing other symptoms. The oil test device described above may be a skin oil tester SM815, which is not limited hereby.

As shown in Figure 2, the skin gloss information acquisition step 201e described above includes determining luster and gloss of the skin texture area by means of a specular gloss test.

In the case that the luster and gloss information is to be acquired, luster and gloss of the skin texture area is determined by means of a specular gloss test. The test method for qualifying a color of the skin currently used by a multi-function skin tester and a skin chroma test probe (MPA9 made in Germany) is to test the change in the color of the skin by a chromaticity system (Lab chromaticity system) defined by the International Commission on Illumination (CIE), a L value (brightness value of the skin), a GLOOSY value (gloss) multi-function skin tester and a skin chroma test probe MPA9 and a GLOOSY probe can qualify the color of the skin accurately, and can reflect the change in gloss of the skin, and enable the gloss of the skin to be qualified more reliably. The surface of an object, gloss of which is indicated as a number, is close to a mirror. The gloss can be evaluated by multiple methods (or instruments), which mainly depends on light source illumination and a viewing angle. The contrast gloss tested by an Ingersoll gloss meter is used for testing gloss of white paper or a near-white paper, luster and gloss is measured by a specular gloss test method, the color of the object depends on a spectral composition of a light source and stimulation of light at various bands reflected on the surface of the object with respect to the human eyes, the stimulation is transferred to cerebral cortex to form a specific feeling, which is color. When the light irradiates onto the skin, absorption for light at different wavelengths by different pigments in the skin are different, which results in a spectral difference of reflected light. White light interacts with the skin, and is converted into colored light by both reflection and absorption, so that the skin exhibits different colors. Influences of different light sources on the color of the skin are different due to different spectra, and human vision is affected by the light irradiating onto a same skin area, so that the observers may have different feelings for the color of the skin. After the light irradiates onto the surface of the skin, or is absorbed by the pigments, or is reflected by the cuticles, about 4% to 8% of the light is reflected by the cuticles, a test indicator L.a.b is coordinate values of the three-dimensional rectangular coordinate system, L represents brightness, the greater the brightness is, the greater the difference of the color is. Furthermore, the gloss on the surface of the skin is reflected by direct reflection and diffuse reflection of the light irradiating onto the surface of the skin. Another device GL200 may be used to specially test the gloss of the surface of the skin, since that the skin is not only different in structure and brightness, but also different in color, thus gloss of different skin can be tested accurately and conveniently by testing the reflected light and scattered light on the surface of the skin. A parallel beam of white light generated by an LED at the top of the probe passes through a planar mirror, and then irradiates onto the surface of the skin at an angle of 60°, a part of light is reflected directly at a same angle as 60° and passes through another planar mirror to irradiate into a receiving sensor. Another part of light is scattered by the surface of the skin, and is then received by a sensor located in a vertical direction of the skin. In this way, the skin gloss testing probe GL200 can not only test light, about the gloss, directly reflected by the skin, but also test the light scattered by the skin, the light tends to be white in the case that the gloss is great, and the light tends to be black in the case that the gloss is small.

202, the acquired skin tone state information, the acquired skin texture elasticity information, the acquired skin moisture information and the acquired luster and gloss information are compared with preset indicator intervals, and the overall skin texture state of the skin texture area is determined by combining with the Qi and Blood state information.

After the Qi and Blood state information is acquired by triggering the steps S1 to S6 of the Qi and Blood information acquisition step 201a in the skin texture area, and the skin tone state information is acquired by triggering the skin tone information acquisition step 201b in the skin texture area, the skin texture elasticity information is acquired by triggering the skin texture information acquisition step 201c in the skin texture area, and the skin moisture information is acquired by triggering the skin moisture information acquisition step 201d in the skin texture area, and the luster and gloss information is acquired by triggering the skin gloss information acquisition step 201e in the skin texture area, the acquired skin tone state information, the acquired skin texture elasticity information, the acquired skin moisture information and the acquired luster and gloss information are compared with preset indicator intervals, and the overall skin texture state of the skin texture area is determined by combining with the Qi and Blood state information.

It should be illustrated that the preset indicator intervals described above include standard data indicators acquired by those skilled in the art through long-term experiments.

The skin texture area described in the embodiment may also be a facial area, and the blood flow distribution information described above may also include local capillary blood flow rate and a local tissue metabolic function of the face.

In the embodiment, the blood flow state of the skin texture area is acquired by the detection device for the Qi and Blood information acquisition step, and the blood flow state is analyzed to obtain the blood flow distribution information corresponding to the blood flow state, whether the blood flow distribution information falls within the preset blood flow information range is judged, to determine the blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired. Also, overall skin texture data information including the Qi and Blood, the skin tone, the skin texture, the skin moisture and the skin luster and gloss is acquired by triggering the skin tone information acquisition step, the skin texture information acquisition step, the skin moisture information acquisition step and the skin gloss information acquisition step, so that the judgement process of processing the acquired skin texture skin is more objective and comprehensive.

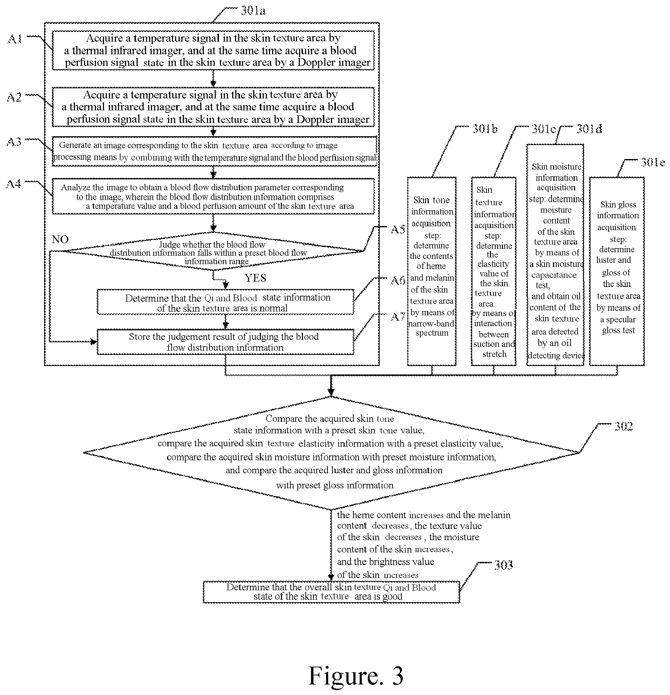

Processes of triggering the skin tone information acquisition step, triggering the skin texture information acquisition step, triggering the skin moisture information acquisition step and triggering the skin gloss information acquisition step are described above in detail. With reference to Figure 3, a method for processing the acquired skin texture data according to another embodiment of the present invention includes steps of:
301, Qi and Blood state information is acquired by triggering a Qi and Blood information acquisition step 301a in a skin texture area, skin tone state information is acquired by triggering a skin tone information acquisition step 301b in the skin texture area, skin texture elasticity information is acquired by triggering a skin texture information acquisition step 301c in the skin texture area, skin moisture information is acquired by triggering a skin moisture information acquisition step 301d in the skin texture area, and luster and gloss information is acquired by triggering a skin gloss information acquisition step 301e in the skin texture area.

In the embodiment, in the case that a skin texture state of a local skin texture area in a certain area is to be determined, the acquired skin texture data are processed. Firstly, the Qi and Blood state information is acquired by triggering the Qi and Blood information acquisition step 301a in a skin texture area, the skin tone state information is acquired by triggering the skin tone information acquisition step 301b in the skin texture area, the skin texture elasticity information is acquired by triggering the skin texture information acquisition step 301c in the skin texture area, the skin moisture information is acquired by triggering the skin moisture information acquisition step 301d in the skin texture area, and the luster and gloss information is acquired by triggering the skin gloss information acquisition step 301e in the skin texture area.

The Qi and Blood state information may be observation for microcirculation of Qi and Blood, including two types of isolated specimen observation and living observation. The isolated specimen observation method mainly includes microvascular perfusion, casting mould, tissue section staining and so on. Microvascular endothelial cell and the ultrastructure thereof can be precisely observed by tissue section, however, a stereo distribution and configuration of the microvessel cannot be shown. The specimen obtained after the perfusion and casting mould can show a three-dimensional configuration of the microvascular well and can measure the length, the width and the area of the microvessel. The disadvantages of the isolated specimen observation are that only microvessel of death or postoperative isolated organs can be used for observation, and the physiological state cannot be reflected. In the living observation, a blood flow speed of the microvessel is observed directly under various microscopes. Currently, there are some shortcomings in the observation method and technology, some observation methods are still invasive, for example, an observation window is installed on the animal skin to expose a subcutaneous vascular network.

The skin tone state information described above may mainly include four pigments of the human skin, that is, black-brown melanin, red oxidized hemoglobin, blue decreased hemoglobin and yellow carotene. From the prospective of skin biology, a color of the human skin is affected by the pigment system, the melanin is a product of melanocytes, content and distribution states of the pigments are important factors for determining the color of the skin. Complexion is pink under the influence of hemoglobin (hemachrome) content in the blood of the skin. Oxygenated hemoglobin is bright red, and (decreased hemoglobin) becomes dark red in hypoxia, the color of the skin changes correspondingly.

The skin texture elasticity information described above may be texture, i.e. skin texture, and may be indicated by a degree of texture, such as skin roughness or smoothness and tenderness. Surface texture of the skin is an important characteristic of skin aging. Skin texture test is to test an elasticity indicator of skin. The method used in a skin texture tester on the market includes a suction method, a torsion method and a method for testing a transmission speed of an elastic shear wave. It should be noted that the skin aging of human includes intrinsic aging and photo-induced aging. The intrinsic aging may be regarded as a change in skin histology and physiology caused by intrinsic genetic factors. The intrinsic aging includes epidermal atrophy, dermal atrophy and flattening of an interface between epidermis and dermis and so on, which, as a performance of natural aging within humans, are irresistible but can be delayed. The photo-induced aging is caused by sunshine, which is a reason for skin keratosis, dark spots, wrinkles and elastic tissue degeneration. Abnormal skin keratosis may include epidermal thickening and densification, which make the skin dry and rough.

The skin moisture information described above may include a skin moisture degree and the oil content, and is indicated as differences in the moisture content and the oil content of the skin cuticle. Moisture is one of the important shaping materials in the cuticle of the skin epidermis, and the cuticle of the epidermis becomes thinner when the skin is aging.

The content of nature moisturizing factor in the cuticle decreases, hydratability of the skin decreases and moisture loss of the skin increases, and a histology structure and morphology change with shrinkage of the cell and atrophy of the tissue, so that small wrinkles gradually occur in the skin, and the surface area of the skin is increasing with further increasing and deepening of the wrinkles, and the epidermis become thinner, moisture loss is more serious and skin aging is aggravated. By testing the moisture of the skin, not only the moisture content in the cuticle of the skin epidermis can be determined directly, but also the extent of the skin aging can be reflected indirectly. The sheddings produced when sebaceous glands secrete oil will clog the pores, therefore, acnes occur. In contrast, sebum secretion decreases, the produced hydro-lipid emulsion decreases, which makes the skin dry, rough, dull, and developing other symptoms.

The luster and gloss information described above may include a brightness change of the skin.

The skin tone state information, the skin texture elasticity information, the skin moisture information and the luster and gloss information will be described in detail in subsequent embodiments, which are not described in detail here anymore.

As shown in Figure 3, the Qi and Blood information acquisition step 301a described above includes steps of:
A1, a temperature signal in the skin texture area is acquired by a thermal infrared imager, and the blood perfusion signal state in the skin texture area is acquired by a Doppler imager.

In the embodiment, in the case that the Qi and Blood state information is acquired by triggering the Qi and Blood information acquisition step 301a, a temperature signal in the skin texture area is firstly acquired by a thermal infrared imager, and at the same time the blood perfusion signal state in the skin texture area is acquired by a Doppler imager. It should be illustrated that an infrared detector detects heat radiation of a body surface, and converts a radiation signal into an infrared image which can be observed by human eyes. The technology has advantages of contactless for human body, no damage or side effect for the human body, a fast detection process and a high test precision, a temperature resolution ranging from 0.01 °C to 0.03 °C. For example, the area of frostbit skin is easily detected by a thermal imager. Since that necrosis occurs in a frostbit area, and no blood is supplied in the frostbit area, the temperature of the frostbit area is lower than that of the surrounding skin. Therefore, the thermal imaging is an indicator for skin Qi and Blood micro-circulation. It can be understood that the infrared imaging is used for acquiring the temperature, and the temperature is realized by the thermotaxic center. The thermotaxic center receives afferent impulses from skin and other areas, and regulates a heat production process and a heat dissipation process, to make the body temperature maintain at a normal level. The heat production process is regulated by increasing tension of the skeletal muscle and chills. The heat loss process is regulated by changing blood flow rate of the skin, for example, by a VARIOSCAN 3021-ST infrared thermal imager.

While the temperature signal in the skin texture area is acquired by the thermal infrared imager, the blood perfusion signal state in the skin texture area is acquired by the Doppler imager, it should be illustrated that an operation principle of the Doppler imager is similar to that of the color ultrasonic Doppler, only ultrasonic wave is changed into laser in the Doppler imager. In the case that a laser beam of a single color interacts with a moving blood cell in the blood flow, light reflected by the moving blood cell in the tissue generates frequency shift in frequency according to Doppler effect principle, the amount of frequency shift is in direct proportion to a moving speed, the intensity of the scattered light is in direct proportion to the number of moving red blood cells. The detector at a laser scanning head can detect these small changes, and process the changes and then perform analysis processing on the processed changes by various image analysis softwares of a computer, to output data on the blood flow state and a curve on a relation between the blood flow and the time, therefore, test and research can be made according to needs in future. It can be understood that the laser described above is scattered after passing through the tissue, and a part of laser is absorbed. A part of scattered laser returns onto a surface of the tissue, and is detected by a detector within the device, the laser signal is converted into microcirculation blood flow of the tissue. After the laser strikes the moving objective according to the Doppler principle for the laser, a wavelength/frequency of the laser is changed, that is, Doppler frequency shift, and a static structure is not changed when being struck by the laser. Intensity and frequency distribution of the laser Doppler frequency shift is in direct proportion to the moving speed of the blood cell in the tissue, and is irrelevant to a moving direction, a blood flow perfusion value can be calculated according to this principle.

Furthermore, the tissue is scanned by a low-energy laser beam based on the Doppler technology for laser by using the PeriScan PIM 3 blood flow perfusion imager, to generate a colored coding micro-circulation blood flow perfusion image. Compared with a dot-mode laser Doppler blood flow imager having a probe, this system cannot study a dynamic change in the blood flow, but can monitor blood flood perfusion data in a wide range, each blood flow perfusion image includes 255×255 monitoring sites, and a monitoring process is contactless, it is not required to be in contact with the monitored object.

It should be illustrated that the principle of the thermal infrared imaging technology described above includes a human body heat production and heat loss mechanism. The human body temperature is regulated by the thermotaxic center. The thermotaxic center receives afferent impulses from skin and other areas, and regulates a heat production process and a heat dissipation process, to make the body temperature maintain at a normal level. The heat production process is regulated by increasing tension of the skeletal muscle and chills. The heat dissipation process is regulated by changing blood flow rate of the skin. Subcutaneous adipose tissue has a tiny small coefficient of thermal conductivity, therefore, the subcutaneous adipose tissue can be served as a thermal insulation system for the body. Heat in deep positions of the body can be conducted to the surface of the skin only by the blood flow. The temperature of the skin of the body surface depends on local blood flow rate and a local tissue metabolism function. The blood circulation of the skin has a characteristic as follows: arteries distributed into the skin passes through an adiabatic system (fat), and forms an arterial rete under a subpapillary layer of the skin, the subcutaneous capillary bends abnormally, and a rich venous plexus is further formed, there are a large number of subcutaneous arteriovenous anastomosis branches. In addition, there is a counter-current mechanism for heat exchange between arteries and veins in deep positions of the body, that is, the vein surrounds the arteries in a network format, in this way, arterial blood having a high blood temperature exchanges heat with venous blood having a low blood temperature, so that the temperature of the arterial blood decreases, and the temperature of the venous blood increases, thereby reducing heat loss. In multiple factors for determining local blood flow rate, a contraction state or a relaxation state of the small artery plays a decisive role, and the contraction state or the relaxation state of the small artery is controlled by regulation of autonomic nervous system. Therefore, in addition to the local blood flow rate and the tissue metabolism, the temperature of the skin of the body surface also reflects a functional state of the autonomic nervous system. In the case that all of three factors of the local blood flow rate, the tissue metabolism and the functional state of the autonomic nervous system described above are abnormal, the abnormality is displayed by the temperature of the body surface, i.e. the temperature of the skin. Therefore, the infrared imaging indicates the Qi and Blood state of the human, a facial Qi and Blood state and a whole-body Qi and Blood state can be indicated by a facial infrared imaging.

A2, a temperature signal in the skin texture area is acquired by a thermal infrared imager, and the blood perfusion signal state in the skin texture area is acquired by a Doppler imager.

In the case that the temperature signal in the skin texture area is acquired by the thermal infrared imager, and the blood perfusion signal state in the skin texture area is acquired by the Doppler imager, a temperature signal in the skin texture area is acquired by the thermal infrared imager, and the blood perfusion signal state in the skin texture area is acquired by the Doppler imager.

A3, an image of the skin texture area is generated according to image processing means in combination with the temperature signal and the blood perfusion signal.

After the temperature signal in the skin texture area is acquired by the thermal infrared imager, and the blood perfusion signal state in the skin texture area is acquired by the Doppler imager, the image of the skin texture area is generated according to image processing means in combination with the temperature signal and the blood perfusion signal. It can be understood that the image described above may be obtained by superimposing an image of the skin texture area corresponding to the temperature signal and an image of the skin texture area corresponding to the blood perfusion signal by those skilled in the art based on the well-known image processing technology.

A4, the image is analyzed to obtain a blood flow distribution parameter corresponding to the image, and blood flow distribution information includes a temperature value and a blood perfusion amount of the skin texture area.

After the image corresponding to the skin texture area is generated based on the blood flow state, the image is analyzed to obtain the blood flow distribution parameter corresponding to the image, the blood flow distribution information includes the temperature value and the blood perfusion amount of the skin texture area.

It should be illustrated that facial temperature values are shown in the Table as follows:

| | M | N | P | Average value |
|---|---|---|---|---|
| Forehead | 36.2 °C | 36.4 °C | 36.3 °C | 36.1 °C |
| Right cheek | 36.5 °C | 36.4 °C | 36.3 °C | 36.7 °C |
| Left cheek | 36.3 °C | 36.3 °C | 36.5 °C | 36.6 °C |
| Lower jaw | 36.1 °C | 36.2 °C | 36.7 °C | 36.4 °C |

Wherein, a facial image is generated based on the body temperature signal in the skin texture area acquired by the thermal infrared imager, and image processing is performed on the facial image to acquire facial imaging sub-areas, the temperature values corresponding to the facial imaging sub-areas are shown in the Table above. For example, for temperature values of three sets M, N, P of facial sub-areas, multiple body temperature signals are acquired in advance, image analysis and processing is performed on the image generated in step A2, to calculate the temperature values corresponding to the sub-areas and the body temperature signals of the sub-areas.

The facial blood flow perfusion amount is shown in the Table as follows:

| | M | N | P | Average value |
|---|---|---|---|---|
| Forehead | 292.3 | 169 | 295.9 | 108.0714 |
| Right cheek | 315.2 | 211.5 | 166.1 | 176.414 |
| Left cheek | 161.4 | 132.3 | 185.6 | 115.943 |
| Lower jaw | 255.9 | 256.8 | 271.4 | 128.671 |

Wherein, a facial image is generated based on the blood perfusion signal in the skin texture area acquired by the Doppler imager, image processing is performed on the facial image to acquire facial imaging sub-areas, and the blood perfusion amount (PU) corresponding to the facial imaging sub-areas is shown in the Table above. For example, for blood flow perfusion amount of three sets M, N and P of facial sub-areas, multiple blood perfusion signals are acquired in advance, and image analysis and processing are performed on the image generated in step A2, to calculate the blood perfusion amount corresponding to the sub-areas and the blood perfusion signals of the sub-areas, an average value of the blood perfusion amount described above may be acquired by capturing the area of the sub-area and then weighting and averaging the captured area, which is not limited hereby.

A5, whether the blood flow distribution information falls within a preset blood flow information range is judged, step A6 is performed in the case that the blood flow distribution information falls within the preset blood flow information range, and step A7 is performed in the case that the blood flow distribution information does not fall within the preset blood flow information range.

In the case that the image is analyzed to obtain blood the flow distribution information corresponding to the image , whether the blood flow distribution information falls within the preset blood flow information range is judged, and step A6 is performed in the case that the blood flow distribution information falls within the preset blood flow information range, and step A7 is performed in the case that the blood flow distribution information does not fall within the preset blood flow information range.

A6, it is determined that the Qi and Blood state information of the skin texture area is normal.

In the case that the blood flow distribution information falls within the preset blood flow information range, it is determined that the Qi and Blood state information of the skin texture area is normal, and step A7 is proceeded.

A7, a judgement result of judging the blood flow distribution information is stored.

In the case that it is determined in step A4 that the blood flow distribution information does not fall within the preset blood flow information range and it is determined that the Qi and Blood state information of the skin texture is normal, a judgement result of judging the blood flow distribution information is stored.

As shown in Figure 3, the skin tone information acquisition step 301b described above includes determining the contents of heme and melanin of the skin texture area by means of narrow-band spectrum.

In the case that the skin tone state information is to be acquired, the contents of heme and melanin of the skin texture area is determined by means of narrow-band spectrum, for example, a skin heme and melanin tester and a test probe (Mexameter MX18) determine the heme and melanin contents in the skin by testing the amount of reflection of light at a specific wavelength on the human skin based on the principle of spectral absorption (RGB). The emitter of an instrument probe emits light at wavelengths of 568 nm, 660 nm and 880 nm to irradiate onto a surface of the skin, and a receiver tests the light reflected by the skin. Since that the amount of emitted light is fixed, the heme and melanin contents can be tested by testing the amount of light absorbed by the skin. A test range of the instrument ranges from 0 to 999, the greater the tested value is, the higher the heme and melanin contents in the skin are.

It should be illustrated that the skin tone described above is closely related to the Qi and Blood. According to technical principle well-known by those skill in the art, in the case that the Qi and Blood run smoothly, the complexion is ruddy and fair, redness increases; and in the case that the Qi and Blood does not run smoothly, the complexion is dark due to Qi stagnation and Blood stasis, and blankness of the skin increases.

As shown in Figure 3, the skin texture information acquisition step 301c described above includes determining the elasticity value of the skin texture area by means of interaction between suction and stretch.

In the case that the skin texture elasticity information is to be acquired, the elasticity value of the skin texture area is determined by means of interaction between suction and stretch. The skin elasticity tester and test probe have a test principle based on suction and tension, negative pressure is forced on the surface of the skin to be tested to suck the skin into a test probe, the depth of skin which is sucked into the test probe is tested by a non-contact optical test system. The test probe includes an optical emitter and an optical receiver, the ratio of the emitted light to the received light is in direct proportion to the depth of the skin which is sucked into the test probe, in this way, a relation curve between an extension length of the skin and time is obtained, and an elasticity performance of the skin can be determined by the curve.

It should be illustrated that the skin texture described above is closely related to the Qi and Blood. According to the technical principle well-known by those skilled in the art, the Qi and Blood may have a function of moistening and nourishing the skin, in the case that the Qi reaches the skin smoothly, the skin has sufficient nutrition, and delicate texture and smoothness, and the texture value of the skin is small.

As shown in Figure 3, the skin moisture information acquisition step 301d described above incudes determining moisture content of the skin texture area by means of a skin moisture capacitance test, and obtaining oil content of the skin texture area detected by an oil detecting device.

In the case that the skin moisture information is to be acquired, the moisture content of the skin texture area is determined by means of a skin moisture capacitance test, and oil content of the skin texture area detected by an oil detecting device is acquired. The world recognized CORNEOMETER capacitance method is used by a skin moisture content tester and test probe (Corneometer CM 825). The principle of the CORNEOMETER capacitance method is that a dielectric constant based on moisture and other substances changes considerably, capacitance of the skin detected by a test capacitor in a proper shape changes with the moisture content, the capacitance of the skin falls within a test range, in this way, the moisture content of the skin can be tested. The result for the moisture content of the skin is represented as a set moisture measurement value (Moisture Measurement Value, MMV). The value of MMV ranges from 0 to 150. The greater the MMV value is, the higher the moisture content of the skin cuticle is. Skin moisture loss TEWL is an important parameter for assessing a function of a skin moisture protection layer, and has been widely accepted internationally. The more complete the skin moisture protection layer is, the higher the moisture content is and the lower a value of the skin moisture loss TEWL is. In other words, a change in moisture vapor pressure on the surface of adjacent skin is measured by the skin moisture loss tester and a test probe (Tewamater TM300) according to the diffusion principle. A relatively stable test microenvironment is formed on the surface of the skin by a specially designed test probe of a cylindrical cavity having two open ends, two sets of temperature sensors and moisture sensors test water vapor pressure gradient between different two points within near epidermis (approximating to 1cm) caused by the moisture loss in the cuticle, to directly test the amount of moisture evaporated via the epidermis, thereby evaluating moisture loss on the surface of the skin. The less the value of TEWL is, the less the amount of moisture loss via the epidermis is, the unit of the amount of moisture loss is g/hm². Furthermore, according to the principle of skin oil secretion, the sheddings produced when the sebaceous glands secrete oil will clog the pores, therefore, acnes occur. In contrast, sebum secretion decreases, the produced hydro-lipid emulsion decreases, which makes the skin dry, rough, dull, and developing other symptoms. The oil test device described above may be a skin oil tester SM815, which is not limited hereby.

It should be illustrated that the skin moisture information described above is closely related to the Qi and Blood. According to the technical principle well-known by those skilled in the art, the Qi and Blood body fluid moistens and nourishes the skin, body fluid and blood share the same source, and the blood can assimilate the body fluid and nourish the skin, in this way, the moisture content in the skin increases, and moisture loss decreases, and oil secretion is moderate.

As shown in Figure 3, the skin gloss information acquisition step 301e described above includes determining luster and gloss of the skin texture area by means of a specular gloss test.

In the case that the luster and gloss information is to be acquired, luster and gloss of the skin texture area is determined by means of a specular gloss test. The test method for qualifying a color of the skin currently used by a multi-function skin tester and a skin chroma test probe (MPA9 made in Germany) is to test the change in the color of the skin by a chromaticity system (Lab chromaticity system) defined by the International Commission on Illumination (CIE), a L value (brightness value of the skin), a GLOOSY value (gloss) multi-function skin tester and a skin chroma test probe MPA9 and a GLOOSY probe can qualify the color of the skin accurately, and can reflect the change in gloss of the skin, and enable the gloss of the skin to be qualified more reliably. The surface of an object, gloss of which is indicated as a number, is close to a mirror. The gloss can be evaluated by multiple methods (or instruments), which mainly depends on light source illumination and a viewing angle. The contrast gloss tested by an Ingersoll gloss meter is used for testing gloss of white paper or a near-white paper, luster and gloss is measured by a specular gloss test method, the color of the object depends on a spectral composition of a light source and stimulation of light at various bands reflected on the surface of the object with respect to the human eyes, the stimulation is transferred to cerebral cortex to form a specific feeling, which is color. When the light irradiates onto the skin, absorption for light at different wavelengths by different pigments in the skin are different, which results in a spectrum difference of reflected light. White light interacts with the skin, and is converted into colored light by both reflection and absorption, so that the skin exhibits different colors. Influences of different light sources on the color of the skin are different due to different spectra, and human vision is affected by the light irradiating onto a same skin area, so that the observers may have different feelings for the color of the skin. After the light irradiates onto the surface of the skin, or is absorbed by the pigments, or is reflected by the cuticles, about 4% to 8% of the light is reflected by the cuticles, a test indicator L.a.b is coordinate values of the three-dimensional rectangular coordinate system, L represents brightness, the greater the brightness is, the greater the difference of the color is. Furthermore, the gloss on the surface of the skin is reflected by direct reflection and diffuse reflection of the light irradiating onto the surface of the skin. Another device GL200 may be used to specially test the gloss of the surface of the skin, since that the skin is not only different in structure and brightness, but also different in color, thus gloss of different skin can be tested accurately and conveniently by testing the reflected light and scattered light on the surface of the skin. A parallel beam of white light generated by an LED at the top of the probe passes through a planar mirror, and then irradiates onto the surface of the skin at an angle of 60°, a part of light is reflected directly at a same angle as 60° and passes through another planar mirror to irradiate into a receiving sensor. Another part of light is scattered by the surface of the skin, and is then received by a sensor located in a vertical direction of the skin. In this way, the skin gloss testing probe GL200 can not only test light, about the gloss, directly reflected by the skin, but also test the light scattered by the skin, the light tends to be white in the case that the gloss is great, and the light tends to be black in the case that the gloss is small.

It should be illustrated that the skin gloss information described above is closely related to the Qi and Blood. According to the technical principle well-known by those skilled in the art, the gloss of the skin is closely related to the Qi and Blood. In the case that the Qi and Blood run smoothly, and circulate well, the skin is glossy and is gorgeous; in contrast, the Qi and Blood cannot reach the skin smoothly, and the skin is dull and is not glossy and not gorgeous.

302, the acquired skin tone state information is compared with a preset skin tone value, the acquired skin texture elasticity information is compared with a preset elasticity value, the acquired skin moisture information is compared with preset moisture information, and the acquired luster and gloss information is compared with preset gloss information. In the case that the heme content increases and the melanin content decreases, the texture value of the skin decreases, the moisture content of the skin increases, and the brightness value of the skin increases, step 303 is performed.

After the Qi and Blood state information is acquired by triggering the steps S1 to S6 of the Qi and Blood information acquisition step 301a in the skin texture area, and the skin tone state information is acquired by triggering the skin tone information acquisition step 301b in the skin texture area, the skin texture elasticity information is acquired by triggering the skin texture information acquisition step 301c in the skin texture area, and the skin moisture information is acquired by triggering the skin moisture information acquisition step 301d in the skin texture area, and the luster and gloss information is acquired by triggering the skin gloss information acquisition step 301e in the skin texture area, the acquired skin tone state information is compared with a preset skin tone value, the acquired skin texture elasticity information is compared with a preset elasticity value, the acquired skin moisture information is compared with preset moisture information, and the acquired luster and gloss information is compared with preset gloss information. In the case that the heme content increases and the melanin content decreases, the texture value of the skin decreases, the moisture content of the skin increases, and the brightness value of the skin increases, step 303 is performed. Furthermore, in the case that the heme content decreases and the melanin content increases, the texture value of the skin increases, the moisture content of the skin decreases, and the brightness value of the skin decreases, the skin texture state is not good or the Qi and Blood state is not good.

It should be illustrated that with reference to the Table as follows, the acquired skin tone state information is compared with the preset skin tone value, the acquired skin texture elasticity information is compared with the preset elasticity value, the acquired skin moisture information is compared with the preset moisture information, and the acquired luster and gloss information is compared with the preset gloss information.

| | Comparison information | Preset comparison value |
|---|---|---|
| Skin tone state information | Melanin content | 65∼352 |
| | Heme content | 151∼468 |
| Skin texture elasticity information | Texture degree of skin | 4.56∼17.09 |
| Skin moisture information | Moisture content | 7.4∼71 |
| Luster and gloss information | L.a.b brightness value | 56.05∼70.82 |

The skin texture area described in the embodiment may also be a facial area, and the blood flow distribution information described above may also include local capillary blood flow rate and a local tissue metabolic function of the face.

303, it is determined that the overall skin texture Qi and Blood state of the skin texture area is good.

After the acquired skin tone state information is compared with the preset skin tone value, the acquired skin texture elasticity information is compared with the preset elasticity value, the acquired skin moisture information is compared with the preset moisture information, and the acquired luster and gloss information is compared with the preset gloss information, and in the case that the heme content increases and the melanin content decreases, the texture value of the skin decreases, the moisture content of the skin increases, and the brightness value of the skin increases, it is determined that the overall skin texture Qi and Blood state of the skin texture area is good.

In the embodiment, the blood flow state of the skin texture is acquired by the detection device for the Qi and Blood information acquisition step, and the blood flow state is analyzed to obtain the blood the flow distribution information corresponding to the blood flow state, whether the blood flow distribution information falls within the preset blood flow information range is judged, to determine the blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired. Also, overall skin texture data information including the Qi and Blood, the skin tone, the skin texture, the skin moisture and the skin luster and gloss is acquired by triggering the skin tone information acquisition step, the skin texture information acquisition step, the skin moisture information acquisition step and the skin gloss information acquisition step, so that the judgement process of processing the acquired skin texture skin is more objective and comprehensive. With the technology for acquiring the blood flow state of the skin texture area by the thermal infrared imager, blood flow rate information of the skin texture area of human can be acquired rapidly, and the blood flow rate information of the skin texture area can be determined directly.

With reference to Figure 4, an apparatus for processing acquired skin texture data according to an embodiment of the present invention includes:
a Qi and Blood module 401 configured to acquire Qi and Blood state information by performing a Qi and Blood information acquisition step in a skin texture area;
a skin tone acquisition module 402 configured to acquire skin tone state information by triggering a skin tone information acquisition step in the skin texture area;
a skin texture acquisition module 403 configured to acquire skin texture elasticity information by triggering a skin texture information acquisition step in the skin texture area;
a moisture acquisition module 404 configured to acquire skin moisture information by triggering a skin moisture information acquisition step in the skin texture area;
a gloss acquisition module 405 configured to acquire luster and gloss information by triggering a skin gloss information acquisition step in the skin texture area;
an analysis module 406 configured to compare the acquired skin tone state information, the acquire skin texture elasticity information, the skin moisture information and the acquired luster and gloss information with preset indicator intervals, and determine the overall skin texture state of the skin texture area by combining with the Qi and Blood state information.

Wherein, the Qi and Blood acquisition module 401 includes:
an acquisition submodule 4011 configured to acquire the blood state of the skin texture area by a detection device;
a generation submodule 4012 configured to generate an image corresponding to the skin texture area based on the blood state;
an analysis submodule 4013 configured to analyze the image to obtain blood flow distribution information corresponding to the image;
a judgement submodule 4014 configured to judge whether the blood flow distribution information falls within a preset blood flow information range, and in the case that the blood flow distribution information falls within the preset blood flow information range, it is determined that the Qi and Blood state information of the skin texture area is normal.

In the embodiment, the Qi and Blood acquisition module 401 acquires the blood flow state of the skin texture by a detection device for the Qi and Blood information acquisition step, the judgement submodule 4014 acquires the blood flow distribution information corresponding to the blood flow state by analyzing the blood flow state, and judges whether the blood flow distribution information falls within the preset blood flow information range, to determine a blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired.

Each module of the apparatus for processing acquired skin texture data is described above in detail, the function of each module will be described below in detail in conjunction with Figure 4, an apparatus for processing acquired skin texture data according to another embodiment of the present invention includes:
a Qi and Blood acquisition module 401 configured to acquire Qi and Blood state information by performing a Qi and Blood information acquisition step in a skin texture area;
a skin tone acquisition module 402 configured to acquire skin tone state information by triggering a skin tone information acquisition step on the skin texture area, specifically, the skin tone acquisition module 402 is configured to determine the contents of heme and melanin of the skin texture area by triggering the skin texture area by means of narrow-band spectrum;
a skin texture acquisition module 403 configured to acquire skin texture elasticity information by triggering a skin texture information acquisition step in the skin texture area, specifically, the skin texture acquisition module 403 is configured to determine the elasticity value of the skin texture area by triggering the skin texture area by means of interaction between suction and stretch;
a moisture acquisition module 404 configured to acquire skin moisture information by triggering a skin moisture information acquisition step in the skin texture area, specifically, the moisture acquisition module 404 is configured to determine moisture content and oil content of the skin texture area by triggering the skin texture area by means of a skin moisture capacitance test;
a gloss acquisition module 405 configured to acquire luster and gloss information by triggering a skin gloss information acquisition step in the skin texture area, specifically, the gloss acquisition module 405 is configured to determine luster and gloss of the skin texture area by triggering the skin texture area by means of a specular gloss test;
an analysis module 406 configured to compare the acquired skin tone state information, the acquired skin texture elasticity information, the acquired skin moisture information and the acquired luster and gloss information with preset indicator intervals, and determine the overall skin texture state of the skin texture area in combination with the Qi and Blood information, specifically, the analysis module 406 is configured to compare the acquired skin tone state information with a preset skin tone value, compare the acquired skin texture elasticity information with a preset elasticity value, compare the acquired skin moisture information with preset moisture information, compare the acquired luster and gloss information with preset gloss information, and in the case that the heme content increases and the melanin content decreases, the texture value of the skin decreases, the moisture content increases and the brightness value of the skin increases, it is determined that the overall skin texture Qi and Blood state of the skin texture area is good.

Wherein, the Qi and Blood acquisition module 401 includes:
an acquisition submodule 4011 configured to acquire the blood state of the skin texture area by a detection device;
a generation submodule 4012 configured to generate an image corresponding to the skin texture area based on the blood state;
an analysis submodule 4013 configured to analyze the image to obtain blood flow distribution information corresponding to the image;
a judgement submodule 4014 configured to judge whether the blood flow distribution information falls within a preset blood flow information range, and determine Qi and Blood state information of the skin texture area is normal in the case that the blood flow distribution information falls within the preset blood flow information range.

In the embodiment, the Qi and Blood acquisition module 401 acquires the blood flow state of the skin texture by a detection device for the Qi and Blood information acquisition step, the judgement submodule 4014 acquires the blood flow distribution information corresponding to the blood flow state by analyzing the blood flow state, and judges whether the blood flow distribution information falls within the preset blood flow information range, to determine a blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired. Also, in the case that the skin tone information acquisition step, the skin texture information acquisition step, the skin moisture information acquisition step and the skin gloss information acquisition step are triggered by the Qi and Blood acquisition module 401, the skin tone acquisition module 402, the skin texture acquisition module 403, the moisture acquisition module 404 and the gloss acquisition module 405, and overall skin texture data information including the Qi and Blood, the skin tone, the skin texture, the skin moisture and the skin luster and gloss is acquired, so that the judgement process of processing the acquired skin texture skin is more objective and comprehensive.

With reference to Figure 5, a system for processing acquired skin texture data according to an embodiment of the present invention includes:
the apparatus 51 for processing acquired skin texture data according to the embodiment as shown in Figure 4, a thermal infrared imaging device 51, a skin hema and melanin test device 53, a skin elasticity test device 54, a skin moisture test device 55, an oil test device 56 and a multi-function skin test device 57.

Wherein, the thermal infrared imaging device 52, the skin hema and melanin test device 53, the skin elasticity test device 54, the skin moisture test device 55, the oil test device 56 and the multi-function skin test device 57 are electrically connected with the apparatus for processing acquired skin texture data, respectively.

It should be illustrated that the thermal infrared imaging device 52 is configured to provide tested Qi and Blood state information to the apparatus for processing the acquired skin texture data via a test probe.

The skin hema and melanin test device 53 is configured to provide tested skin tone state information to the apparatus 51 for processing the acquired skin texture data via a test probe.

The skin elasticity test device 54 is configured to provide tested skin texture elasticity information to the apparatus 51 for processing the acquired skin texture data via a test probe.

The skin moisture test device 55 is configured to provide tested skin moisture information to the apparatus 51 for processing the acquired skin texture data via a test probe.

The oil test device 56 is configured to provide tested skin oil content information to the apparatus for processing the acquired skin texture data via a test probe.

The multi-function skin test device 57 is configured to provide tested luster and gloss information to the apparatus 51 for processing the acquired skin texture data via a test probe.

In the embodiment, in the case that the apparatus 51 for processing the acquired skin texture data are connected with the thermal infrared imaging device 52, the blood flow state of the skin texture area is acquired by the detection device for the Qi and Blood information acquisition step, and blood flow distribution information corresponding to the blood flow state is acquired by analyzing the blood flow state, whether the blood flow distribution information falls within the preset blood flow information range is judged, to determine the blood flow state, thereby solving the technical problem in the prior art of misjudgement of the skin texture state due to the fact that the skin texture state can be determined only by acquiring the temperature of the skin, and the most critical information data of the "Qi and Blood" of the skin cannot be directly acquired. Also, the skin hema and melanin test device 53, the skin elasticity test device 54, the skin moisture test device 55, the oil test device 56 and the multi-function skin test device 57 are electrically connected with the apparatus 51 for processing the acquired skin texture data, respectively, therefore, overall skin texture data information including the Qi and Blood, the skin tone, the skin texture, the skin moisture and the skin luster and gloss is acquired by triggering the skin tone information acquisition step, the skin texture information acquisition step, the skin moisture information acquisition step and the skin gloss information acquisition step, so that the judgement process of processing the acquired skin texture skin is more objective and comprehensive.

It can be clearly understood by those skilled in the art that for convenience and ease of description, the specific operation processes of the system, apparatus and unit described above may refer to corresponding processes in the method embodiments described above, which are not described repeatedly anymore.

In the embodiments provided by the present application, it should be understood that the disclosed system, apparatus and method may be implemented in other ways. For example, the device embodiments described above are only schematic. For example, the units or modules are divided based on a logic function thereof, and they may be divided in another way in practice. For example, multiple units or modules may be combined or integrated into another system, or some features may be omitted or not performed. In addition, a coupling, a direct coupling or a communication connection between displayed or discussed constitutional components may be an indirect coupling or a communication connection via some interfaces, devices or units, and may be in an electrical form, a mechanical form or another form.

A unit described as a separate component may be or may not be physically separated in, a component displayed as a unit may be or may not be a physical unit, that is, may be placed in a same position or may be distributed in multiple network units. A part of or all modules may be selected if desired to realize the object of the embodiments.

In addition, all function units according to the embodiment of the present invention may be integrated into one processing unit, or may be a physically separate unit, or two or more units are integrated into one unit. The integrated unit described above may be realized in hardware, or may be realized by a software function unit.

The integrated unit may be stored in a computer readable storage medium if the integrated unit is implemented in a software function unit and sold or used as a separate product. Base on such understanding, the essential part of the technical solution of the present invention or the part of the technical solution of the present invention contributed to the prior art or all of or a part of the technical solution may be embodied in a software product. The computer software product is stored in a storage medium, which includes several instructions to make a computer device (may be a personal computer, a server, a network device or the like) execute all or a part of the steps of the method according to the embodiment of the present application. The storage medium described above includes various mediums which can store program codes, such as a USB disk, a mobile hard disk, a read-only memory (ROM), a random access memory (RAM), a disk and a optical disc.

As described above, the above embodiments are merely provided for describing the technical solutions of the present invention, but are not intended to limit the present invention. Although the present invention is described in detail with reference to the foregoing embodiments, those skilled in the art should understand that, they can still modify technical solutions described in the foregoing embodiments, or make equivalent substitutions to a part or all of the technical features; and such modifications or substitutions do not enable the essence of corresponding technical solutions to depart from the spirit and scope of the technical solutions according to the embodiments of the present invention.

## Claims

1. A method for processing acquired skin texture data, comprising:
acquiring Qi and Blood state information by triggering a Qi and Blood information acquisition step in a skin texture area, acquiring skin tone state information by triggering a skin tone information acquisition step in the skin texture area, acquiring skin texture elasticity information by triggering a skin texture information acquisition step in the skin texture area, acquiring skin moisture information by triggering a skin moisture information acquisition step in the skin texture area, and acquiring luster and gloss information by triggering a skin gloss information acquisition step in the skin texture area; and
comparing the acquired skin tone state information, the acquired skin texture elasticity information, the acquired skin moisture information and the acquired luster and gloss information with preset indicator intervals, and determining the overall skin texture state of the skin texture area by combining with the Qi and Blood state information;
wherein the Qi and Blood information acquisition step comprises:
acquiring the blood state of the skin texture area by a detection device;
generating an image corresponding to the skin texture area based on the blood state;
analyzing the image to obtain blood flow distribution information corresponding to the image; and
judging whether the blood flow distribution information falls within a preset blood flow information range, and in the case that the blood flow distribution falls within the preset blood flow information range, it is determined that the Qi and Blood state information of the skin texture area is normal.

2. The method for processing acquired skin texture data according to claim 1, wherein said acquiring the blood state of the skin texture area by a detection device, generating an image corresponding to the skin texture area based on the blood flow state specifically comprises:
acquiring a temperature signal in the skin texture area by a thermal infrared imager, and at the same time acquiring the blood perfusion signal state in the skin texture area by a Doppler imager; and
generating the image corresponding to the skin texture area according to image processing means in combination with the temperature signal and the blood perfusion signal.

3. The method for processing acquired skin texture data according to claim 2, wherein said analyzing the image to obtain blood flow distribution information corresponding to the image specifically comprises:
analyzing the image to obtain a blood flow distribution parameter corresponding to the image, wherein the blood flow distribution information comprises a temperature value and a blood perfusion amount of the skin texture area.

4. The method for processing acquired skin texture data according to claim 1, wherein the skin tone information acquisition step specifically comprises:
determining the contents of heme and melanin of the skin texture area by means of narrow-band spectrum.

5. The method for processing acquired skin texture data according to claim 1, wherein the skin texture information acquisition step specifically comprises:
determining the elasticity value of the skin texture area by means of interaction between suction and stretch.

6. The method for processing acquired skin texture data according to claim 1, wherein the skin moisture information acquisition step specifically comprises:
determining moisture content of the skin texture area by means of a skin moisture capacitance test, and obtaining oil content detected by an oil detecting device.

7. The method for processing acquired skin texture data according to claim 1, wherein the skin gloss information acquisition step specifically comprises:
determining luster and gloss of the skin texture area by means of a specular gloss test.

8. The method for processing acquired skin texture data according to any one of claims 1 to 7, wherein said comparing the acquired skin tone state information, the acquired skin texture elasticity information, the acquired skin moisture information and the acquired luster and gloss information with preset indicator intervals, and determining the overall skin texture state of the skin texture area by combining with the Qi and Blood state information specifically comprises:
comparing the acquired skin tone state information with a preset skin tone value, comparing the acquired skin texture elasticity information with a preset elasticity value, comparing the acquired skin moisture information with preset moisture information, comparing the acquired luster and gloss information with preset gloss information, and in the case that the heme content increases and the melanin content decreases, the texture value of the skin decreases, the moisture content of the skin increases, and the brightness value of the skin increases, it is determined that the overall skin texture Qi and Blood state of the skin texture area is good.

9. The method for processing acquired skin texture data according to any one of claims 1 to 7, wherein the skin texture area is a facial area.

10. The method for processing acquired skin texture data according to any one of claims 1 to 7, wherein the blood flow distribution information comprises local capillary blood flow rate and local tissue metabolic function of the face.

11. An apparatus for processing acquired skin texture data, comprising:
a Qi and Blood acquisition module configured to acquire Qi and Blood state information by performing a Qi and Blood information acquisition step in a skin texture area;
a skin tone acquisition module configured to acquire skin tone state information by triggering a skin tone information acquisition step in the skin texture area;
a skin texture acquisition module configured to acquire skin texture elasticity information by triggering a skin texture information acquisition step in the skin texture area;
a moisture acquisition module configured to acquire skin moisture information by triggering a skin moisture information acquisition step in the skin texture area;
a gloss acquisition module configured to acquire luster and gloss information by triggering a skin gloss information acquisition step in the skin texture area; and
an analysis module configured to compare the acquired skin tone state information, the acquired skin texture elasticity information, the acquired skin moisture information and the acquired luster and gloss information with preset indicator intervals, and determine the overall skin texture state of the skin texture area by combining with the Qi and Blood state information;
wherein the Qi and Blood acquisition module specifically comprises:
an acquisition submodule configured to acquire the blood state of the skin texture area by a detection device;
a generation submodule configured to generate an image corresponding to the skin texture area based on the blood state;
an analysis submodule configured to analyze the image to obtain blood flow distribution information corresponding to the image; and
a judgement submodule configured to judge whether the blood flow distribution information falls within a preset blood flow information range, and in the case that the blood flow distribution information falls within the preset blood flow information range, it is determined that the Qi and Blood state information of the skin texture area is normal.

12. The apparatus for processing acquired skin texture data according to claim 11, wherein
the skin tone acquisition module is specifically configured to determine the contents of heme and melanin of the skin texture area by triggering the skin texture area by means of narrow-band spectrum;
the skin texture acquisition module is specifically configured to determine the elasticity value of the skin texture area by triggering the skin texture area by means of interaction between suction and stretch;
the moisture acquisition module is specifically configured to determine the moisture content of the skin texture area by triggering the skin texture area by means of a skin moisture capacitance test, and obtain the oil content detected by an oil detecting device; and
the gloss acquisition module is specifically configured to determine the luster and gloss of the skin texture area by triggering the skin texture area by means of a specular gloss test.

13. The apparatus for processing acquired skin texture data according to claim 11, wherein the analysis module is specifically configured to compare the acquired skin tone state information with a preset skin tone value, compare the acquired skin texture elasticity information with a preset elasticity value, compare the acquired skin moisture information with preset moisture information, and compare the acquired luster and gloss information with preset gloss information, and in the case that the heme content increases and the melanin content decreases, the texture value of the skin decreases, the moisture content of the skin increases, and the brightness value of the skin increases, it is determined that the overall skin texture Qi and Blood state of the skin texture area is good.

14. A system for processing acquired skin texture data, comprising:
the apparatus for processing acquired skin texture data according to any one of claims 11 to 13; and
a thermal infrared imaging device, a skin heme and melanin test device, a skin elasticity test device, a skin moisture test device, an oil test device and a multi-function skin test device;
wherein the thermal infrared imaging device, the skin heme and melanin test device, the skin elasticity test device, the skin moisture test device, the oil test device and the multi-function skin test device are electrically connected with the apparatus for processing acquired skin texture data, respectively.

15. The system for processing acquired skin texture data according to claim 14, wherein
the thermal infrared imaging device is configured to provide tested Qi and Blood state information to the apparatus for processing acquired skin texture data via a test probe;
the skin heme and melanin test device is configured to provide tested skin tone state information to the apparatus for processing acquired skin texture data via a test probe;
the skin elasticity test device is configured to provide tested skin texture elasticity information to the apparatus for processing acquired skin texture data via a test probe;
the skin moisture test device is configured to provide tested skin moisture information to the apparatus for processing acquired skin texture data via a test probe;
the oil test device is configured to provide tested skin oil content information to the apparatus for processing acquired skin texture data via a test probe; and
the multi-function skin test device is configured to provide tested luster and gloss information to the apparatus for processing acquired skin texture data via a test probe.
